# EUROPEAN PATENT SPECIFICATION

(11) **EP 4 427 663 B1**
(45) Date of publication and mention of the grant of the patent: **14.01.2026**
(21) Application number: 22910632.3
(22) Date of filing: 08.11.2022
(51) Int. Cl.: A61B 3/024

(54) **VISUAL FIELD TESTING APPARATUS**
SICHTSFELDTESTGERÄT
APPAREIL DE TEST DU CHAMP VISUEL

(30) Priority: 24.12.2021 JP 2021210070
(43) Date of publication of application: 11.09.2024
(73) Proprietor: FINDEX Inc., Chiyoda-ku, Tokyo 100-0004 (JP)
(72) Inventor: AIBARA Teruo, Tokyo 100-0004 (JP)
(74) Representative: Hasegawa, Kan
(86) International application number: PCT/JP2022/041472
(87) International publication number: WO 2023/119913

(56) References cited:
- WO-A1-2019/050048
- AU-A1- 2015 326 644
- JP-A- 2011 161 122
- JP-A- 2020 141 848
- US-A1- 2019 200 858
- US-B1- 6 290 357

## Description

### TECHNICAL FIELD

The present invention relates to a visual field testing apparatus.

### BACKGROUND ART

Visual field testing apparatuses for testing visual field have been provided conventionally; for example, there is such a disclosure at Patent Reference No. 1, below. At the visual field testing apparatus disclosed at Patent Reference No. 1, below, a user is made to use a button or other such input apparatus to input whether or not a target has been seen by his or her eye and recognized.

However, depending on the user, there have been cases in which the user is unfamiliar with operation of the input apparatus, or due to tension or the like is unable to skillfully operate the input apparatus, and so fails to properly carry out input therewith, as a result of which there has been inability to accurately carry out visual field testing.

In contradistinction thereto, a visual field testing apparatus is disclosed at Patent Reference No. 2, below, in which the line of sight which is the direction in which a user is looking is automatically detected by a line of sight detection apparatus, and determination is automatically made as to whether or not the user visually recognizes the target.

### PRIOR ART REFERENCES

### PATENT REFERENCES

Patent Reference No. 1: WO 2017-022757
Patent Reference No. 2: Japanese Patent Application Publication Kokai No. 2011-161122

JP 2020-141848 A discloses a system for automatically and determining whether a user is visually recognizing a visual target or visual mark displayed on a display. The system includes a display, a memory for gaze logs, and a recognition judgment unit. The system is configured to judge recognition based on gaze proximity and fixation duration, and may use a virtual magnified sphere for collision detection to account for gaze detection errors. The system is implemented in a head-mounted display for visual field testing.

AU 2015326644 A1 discloses a method, apparatus, and software for testing a visual field using a display, eye tracker, and a movable marker indicating the patient's gaze. The system presents fixation and new targets, tracks gaze, and records detections. The apparatus and method perform calibration, visual/audible notifications, reaction time recording, repositioning targets, tolerance paths, and optional patient-actuated indicators. The apparatus may be head-mounted or use conventional displays.

### SUMMARY OF INVENTION

### PROBLEM TO BE SOLVED BY INVENTION

Here, visual field testing ordinarily takes 10 minutes or more, and where-as is the case with the visual field test apparatus disclosed at Patent Reference No. 2-visual recognition determination is carried out automatically, it is necessary for the user who is the test subject to constantly be made to gaze continuously at targets that are sequentially displayed at a display during this period.

For this reason, the user is continuously made to experience a constant state of stress during visual field testing, and the burden placed on the user in connection with testing is extremely large. For this reason, among children and the elderly, for whom it may be difficult to maintain concentration for long periods of time, there are cases in which gazing, which was supposed to have been for a prescribed amount of time, has been discontinued partway through, preventing accurate testing from being carried out.

The present invention was conceived in light of such problems, it being an object thereof to provide a visual field testing apparatus such as will reduce the burden placed on the user in accompaniment to visual field testing and permit more accurate visual field testing to be carried out.

### MEANS FOR SOLVING PROBLEM

To solve the foregoing problems, in the context of a visual field testing apparatus for testing a range of visual field of a user, a visual field testing apparatus associated with the present invention is provided by claim 1. It is characterized in that it comprises a display for displaying targets; a line of sight detection unit that detects a line of sight of the user and that outputs line of sight information pertaining to a direction of the line of sight; a visual recognition determination unit that carries out determination as to whether or not the user has visually recognized the targets based on the line of sight information and position information of the targets; and a visual field testing unit that sequentially displays the targets at prescribed locations at the display and that carries out testing of the range of visual field based on output from the visual recognition determination unit; wherein the visual field testing unit comprises a visual recognition detection feedback unit which, when it is detected due to the output from the visual recognition determination unit that the user has visually recognized the target which is currently displayed at the display, carries out feedback processing for a prescribed time to let the user know that detection has occurred, wherein the time during which the feedback processing is carried out is between 200 ms and 800 ms, wherein the visual recognition detection feedback unit is such that the feedback processing takes the form of causing the target which is currently displayed at the display and which has been visually recognized to change color or shape, wherein the visual field testing unit is such that, together with conclusion of the feedback processing, the target which has been visually recognized is made to disappear, and the next target is displayed at the display.

### BENEFIT OF INVENTION

By providing feedback with respect to detection of visual recognition of targets by a user, the present invention allows the user to be released even for a short time from the state of stress, makes it possible for the user to better concentrate and gaze at targets, and permits more accurate visual field testing to be carried out.

### BRIEF DESCRIPTION OF DRAWINGS

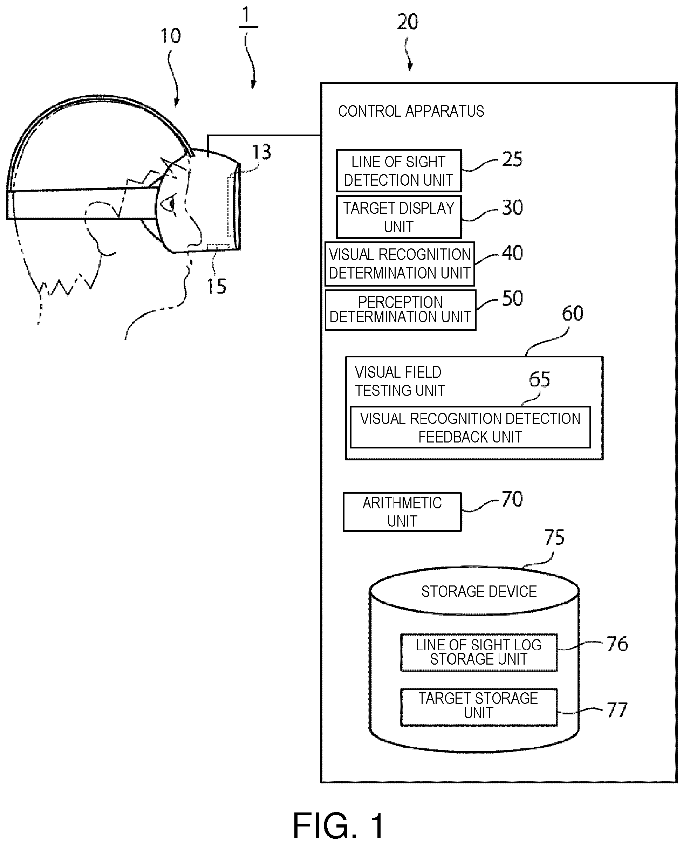

[FIG. 1] FIG. 1 is a schematic diagram showing in simplified fashion the constitution of a target testing apparatus associated with an embodiment of the present invention.
[FIG. 2] FIG. 2 is a drawing for describing visual recognition detection feedback processing associated with an embodiment of the present invention.
[FIG. 3] FIG. 3 is a drawing for describing a variation on visual recognition detection feedback processing associated with an embodiment of the present invention.
[FIG. 4] FIG. 4 is a drawing for describing a variation on visual recognition detection feedback processing associated with an embodiment of the present invention.

### EMBODIMENTS FOR CARRYING OUT INVENTION

Visual field testing apparatus 1, which is associated with an embodiment of the present invention, will be described in detail hereinbelow with reference to the drawings. It is characteristic of the present embodiment that when a user who is a test subject actually sees and visually recognizes targets that are sequentially displayed at display 13 during visual field testing, feedback from the apparatus lets the user know that said visual recognition has been detected.

Visual field testing apparatus 1 comprises head mounted display (HMD) 10, and control apparatus 20 which is a computer connected by a communication cable to HMD 10. HMD 10, which is equipment that is mounted on the head of the user during visual field testing, comprises display 13 which displays targets, camera 15 for capturing images of the eyes of the user, and so forth.

HMD 10 is equipped with line of sight detection functionality by which a line of sight that is a direction in which the user on whom HMD 10 is mounted is looking with his or her eye(s) is automatically detected in cooperation with line of sight detection unit 25, described below. Display 13 and camera 15 respectively have units installed thereat for the right eye and the left eye.

Control apparatus 20 comprises central processing unit(s) (CPU) or other such arithmetic unit(s) 70 for carrying out various types of operations, as well as hard disc drive(s) (HDD) for storing various types of information, and/or random access memory or memories (RAM) capable of being used as a work area during arithmetic processing, or other such storage device(s) 75.

Storage device 75 comprises line of sight log storage unit 76 which sequentially records line of sight information detected by line of sight detection unit 25, described below; and target storage unit 77 which records target information pertaining to targets to be displayed at display 13.

Furthermore, control apparatus 20 comprises-in terms of functionalities-line of sight detection unit 25, target display unit 30, visual recognition determination unit 40, perception (visibility) determination unit 50, and visual field testing unit 60, these functionalities being implemented by causing prescribed program(s) stored at storage device 75 to be executed by arithmetic unit 70.

Line of sight detection unit 25 detects the direction in which the user is looking, i.e., the line of sight, based on captured image(s) of the eye(s) of the user which is the output of camera 15. More specifically, line of sight detection unit 25 outputs two angular components (θ, ϕ) which are polar coordinates (spherical coordinates) in the form of line of sight information (line of sight angle) indicating the direction of the line of sight. This line of sight detection is carried out independently for the right eye and the left eye.

Target display unit 30 causes white targets of prescribed size to be sequentially displayed at prescribed sensitivity (brightness) at prescribed coordinate locations at display 13 based on target information recorded at target storage unit 77.

Visual recognition determination unit 40 carries out determination as to whether or not the target has actually been seen and visually recognized by the user, based on the line of sight detected by line of sight detection unit 25 and the coordinates of the target displayed at display 13. More specifically, visual recognition determination is carried out based on whether or not an imaginary line that is an extension in the direction of the line of sight detected by line of sight detection unit 25 within a prescribed coordinate system would physically collide with the target in question. In accordance with the present embodiment, a determination will be made that there has been a collision and that visual recognition has taken place if upon comparison of the line of sight angle detected by line of sight detection unit 25 and the target angle indicating the location of the target in question, the angular difference therebetween is within 2°.

Note that it is also possible for visual recognition determination unit 40 to cause visual recognition determination to be carried out using logical determinative techniques that make use of logged line of sight information. For example, a constitution may be adopted in which visual recognition determination unit 40 causes logical determination of visual recognition to be carried out when the line of sight approaches the target in question and continues to be located near said target.

Perception determination unit 50 carries out determination as to whether or not a target at a position other than a position which is in the line of sight of the user has been seen by (is visible to) the user, i.e., whether or not it has been perceived. Perception determination unit 50 might, for example, be such that after the target has been displayed at display 13, determination as to whether perception occurred at the time that the target was displayed is carried out based on the arrival path which is the path taken by the line of sight, and the arrival time which is the time taken by the line of sight, in going from the position that the line of sight was in at the time that display occurred until it arrives (is visually perceived) at said target.

Visual field testing unit 60 controls line of sight detection unit 25, target display unit 30, visual recognition determination unit 40, and perception determination unit 50, and causes visual field testing to be carried out. Visual field testing unit 60 is equipped with visual recognition detection feedback unit 65 which, when it is detected due to output from visual recognition determination unit 40 that the user has visually recognized a target displayed at display 13 during visual field testing, lets the user know that visual recognition has been detected.

Visual recognition detection feedback unit 65 controls target display unit 30, and carries out processing to let the user know that detection of visual recognition has occurred by changing the color of the target displayed at display 13 from the standard white color to another color.

In accordance with the present embodiment, visual recognition detection feedback unit 65 carries out feedback processing by, 500 ms after detection of visual recognition has occurred, changing the color of the target to green for a period of 300 ms. Together with the conclusion of feedback processing, the target that was visually recognized is-while still green-made to disappear, and the next target is promptly displayed in the standard white color at display 13.

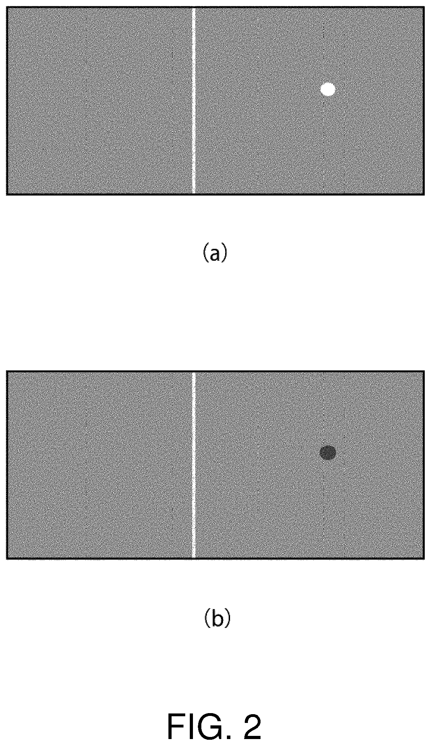

(a) at FIG. 2 shows a situation in which a target is displayed in the standard white color at display 13; (b) at FIG. 2 shows a situation in which a target is displayed in green at display 13.

How many second(s) after detection of visual recognition has occurred feedback processing should be carried out, the time during which feedback processing should be carried out, the timing with which a target that has been visually recognized should be made to disappear, the timing with which the next target should be displayed, and so forth may of course be varied as appropriate. Note further that, to maintain a time capable of being noticed by the user, while also not causing visual field testing time to be too long, the time during which feedback processing is carried out be 200 ms to 800 ms.

Furthermore, regarding the color to which the target is changed for feedback processing, this is not limited to green, it being possible to employ blue, red, or any other color as appropriate such as will readily allow the user to be made aware of the change in color.

During visual field testing, because the user who is the test subject is instructed to continue to gaze at the targets displayed at display 13, he or she is continuously in a state of stress throughout testing, and so there are situations in which, when concentration is interrupted partway therethrough and it becomes impossible to continue to follow the targets, reduction in the accuracy with which testing is carried out can occur.

To address this, in accordance with the present embodiment, notwithstanding that it is only for an extremely short time until the next target is displayed, the fact that feedback is provided to let the user know that visual field testing apparatus 1 has detected that the user has visually recognized the target allows the user to be released from the state of stress, which makes it dramatically easier to maintain the concentration necessary for testing, as a result of which it is possible to further increase the accuracy of testing.

Furthermore, the change in color of the target is easily noticed by the user as he or she continues to look at the target; and furthermore, as this requires no addition of some member or the like, it being possible to easily implement this through modification of program(s), making it possible for great benefit to be provided.

Above, description has been given with respect to visual field testing apparatus 1 associated with the present embodiment; in accordance with the present embodiment, by providing feedback to let the user know that visual field testing apparatus 1 has detected that the user has visually recognized the target, it is possible to cause visual field testing to be carried out with increased accuracy.

Description will next be given with respect to a variation on the processing by which visual recognition detection feedback is given to the user by visual recognition detection feedback unit 65. Whereas in the foregoing embodiment feedback was provided by changing the color of the target displayed at display 13 for a prescribed time, it is also for example possible to provide feedback by changing the shape of the target, or by changing the combination of both the color and shape of the target.

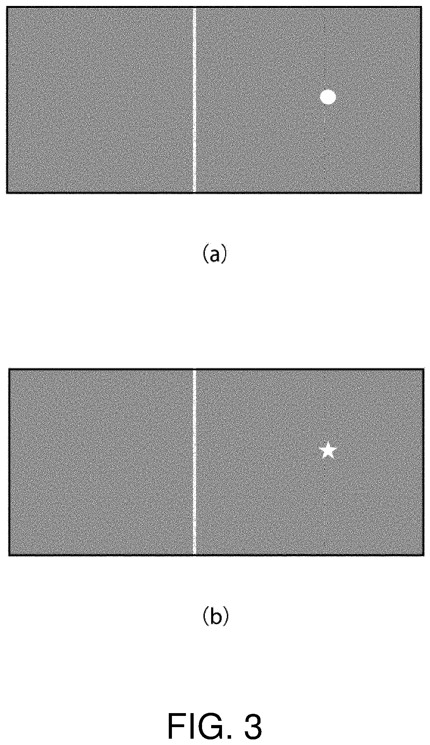

FIG. 3 is a drawing showing an example of how the shape of the target might be changed so as to provide feedback. (a) at FIG. 3 shows a situation in which a target is displayed with the standard circular shape at display 13; (b) at FIG. 3 shows a situation in which a target is displayed in the shape of a star at display 13. Thus, a target that would be standardly displayed with circular shape may be made to be in the shape of a star during the period when feedback is being provided. Feedback may of course be provided not only by causing the shape thereof to be changed to that of a star, but also by causing the shape thereof to be changed to that of a square or other such polygon or other such shape as appropriate.

Furthermore, as visual method(s) for providing feedback with respect to detection of visual recognition, besides changing the color and/or shape of the target, it is also possible to change the color of the background at display 13 and/or to cause a symbol or text indicating that detection has occurred to be displayed at prescribed location(s) at display 13.

Moreover, as method(s) for providing feedback with respect to detection of visual recognition, besides methods in which this is made known visually, it is also possible to make this known to the user tactilely, auditorily, and/or by way of other such sense(s). Where feedback is to be provided to the user tactilely, a small vibrating element might be mounted on HMD 10, and the vibrating element might be made to vibrate for a prescribed time (e.g., for 300 ms) while feedback is being provided.

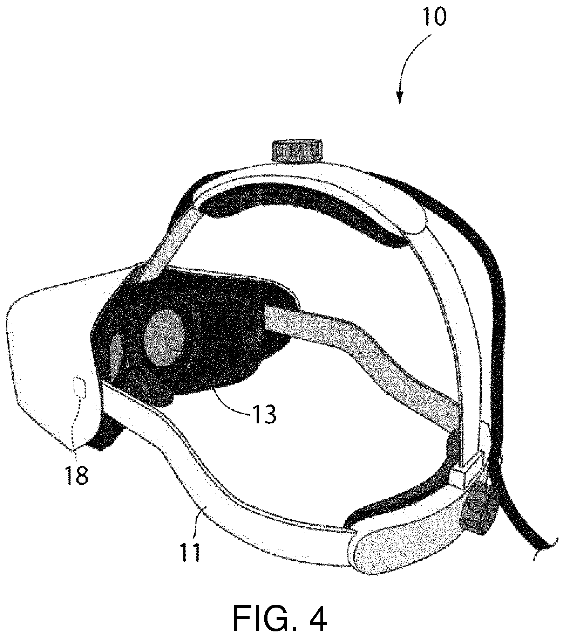

FIG. 4 is a schematic diagram showing in simplified fashion the constitution of HMD 10 in a situation in which tactile feedback is provided to the user. As shown in same drawing, by installing a small vibrating element 18 at the interior on either side of case 11 of HMD 10, it will be possible to provide the user with feedback without disturbing the way in which HMD 10 is mounted on the user.

Furthermore, where feedback is to be provided auditorily, by causing speaker(s), not shown, at visual field testing apparatus 1 to produce an audible notification, it will be possible to let the user know that visual recognition has been detected. It is of course also possible to carry out feedback processing by providing a combination of visual, tactile, auditory, and/or other such feedback.

While embodiments of the present invention have been described above, embodiments for carrying out the present invention are not limited to the foregoing embodiments, a great many variations being possible without departing from the scope of the present invention. For example, whereas an HMD that is mounted on the head was employed as display, it is also possible to employ an ordinary liquid crystal monitor of the standalone or wall-mounted type or the like.

### EXPLANATION OF REFERENCE NUMERALS

- 1: Visual field testing apparatus
- 10: HMD
- 11: Case
- 13: Display
- 15: Camera
- 18: Vibrating element
- 20: Control apparatus
- 25: Line of sight detection unit
- 30: Target display unit
- 40: Visual recognition determination unit
- 50: Perception determination unit
- 60: Visual field testing unit
- 65: Visual recognition detection feedback unit
- 70: Arithmetic unit
- 75: Storage device
- 76: Line of sight log storage unit
- 77: Target storage unit

## Claims

1. A visual field testing apparatus (1) for testing a range of visual field of a user comprising:
a display (13) for displaying targets;
a line (25) of sight detection unit that detects a line of sight of the user and that outputs line of sight information pertaining to a direction of the line of sight;
a visual recognition determination unit (40) that carries out determination as to whether or not the user has visually recognized the targets based on the line of sight information and position information of the targets; and
a visual field testing unit (60) that sequentially displays (13) the targets at prescribed locations at the display (13) and that carries out testing of the range of visual field based on output from the visual recognition determination unit (40);
wherein the visual field testing unit (60) comprises a visual recognition detection feedback unit (65) which, when it is detected due to the output from the visual recognition determination unit (40) that the user has visually recognized the target which is currently displayed at the display (13), carries out feedback processing for a prescribed time to let the user know that detection has occurred, wherein the time during which the feedback processing is carried out is between 200 ms and 800 ms, wherein the visual recognition detection feedback unit (65) is such that the feedback processing takes the form of causing the target which is currently displayed at the display (13) and which has been visually recognized to change color or shape, wherein the visual field testing unit (60) is such that, together with conclusion of the feedback processing, the target which has been visually recognized is made to disappear, and the next target is displayed at the display (13).

## Patentansprüche

1. Gesichtsfeldprüfvorrichtung (1) zum Prüfen eines Bereichs eines Gesichtsfeldes eines Benutzers, umfassend:
eine Anzeige (13) zum Anzeigen von Zielen;
eine Sichtlinien (25) erfassungseinheit, die eine Sichtlinie des Benutzers erfasst und Sichtlinieninformationen ausgibt, die sich auf eine Richtung der Sichtlinie beziehen;
eine visuelle Erkennungsbestimmungseinheit (40), die eine Bestimmung, ob der Benutzer die Ziele visuell erkannt hat oder nicht, basierend auf den Sichtlinieninformationen und Positionsinformationen der Ziele, durchführt; und
eine Gesichtsfeldprüfeinheit (60), die die Ziele an vorgegebenen Stellen an der Anzeige (13) nacheinander anzeigt (13) und die ein Prüfen des Bereichs des Gesichtsfelds basierend auf einer Ausgabe von der visuellen Erkennungsbestimmungseinheit (40) durchführt;
wobei die Gesichtsfeldprüfeinheit (60) eine visuelle Erkennungserfassungsrückmeldeeinheit (65) umfasst, die, wenn aufgrund der Ausgabe von der visuellen Erkennungsbestimmungseinheit (40) erfasst wird, dass der Benutzer das aktuell an der Anzeige (13) angezeigte Ziel visuell erkannt hat, eine Rückmeldeverarbeitung für eine vorgegebene Zeit durchführt, um den Benutzer wissen zu lassen, dass eine Erfassung stattgefunden hat, wobei die Zeit, während der die Rückmeldeverarbeitung durchgeführt wird, zwischen 200 ms und 800 ms liegt, wobei die visuelle Erkennungserfassungsrückmeldeeinheit (65) derart ist, dass die Rückmeldeverarbeitung die Form annimmt, zu bewirken, dass das Ziel, das aktuell an der Anzeige (13) angezeigt wird und das visuell erkannt wurde, seine Farbe oder Form ändert, wobei die Gesichtsfeldprüfeinheit (60) derart ist, dass zusammen mit dem Abschluss der Rückmeldeverarbeitung das Ziel, das visuell erkannt wurde, verschwinden gelassen wird und das nächste Ziel an der Anzeige (13) angezeigt wird.

## Revendications

1. Appareil de test de champ visuel (1) pour tester une plage de champ visuel d'un utilisateur comprenant :
un dispositif d'affichage (13) destiné à afficher des cibles ;
une unité de détection de ligne (25) de visée qui détecte une ligne de visée de l'utilisateur et qui délivre en sortie des informations de ligne de visée relatives à une direction de la ligne de visée ;
une unité de détermination de reconnaissance visuelle (40) qui effectue une détermination pour savoir si oui ou non l'utilisateur a reconnu visuellement les cibles sur la base des informations de ligne de visée et des informations de position des cibles ; et
une unité de test de champ visuel (60) qui affiche séquentiellement (13) les cibles au niveau des emplacements prescrits sur le dispositif d'affichage (13) et qui effectue un test de la plage de champ visuel sur la base de la sortie provenant de l'unité de détermination de reconnaissance visuelle (40) ;
ladite unité de test de champ visuel (60) comprenant une unité de rétroaction de détection de reconnaissance visuelle (65) qui, lorsqu'en raison de la sortie provenant de l'unité de détermination de reconnaissance visuelle (40) est détectée la reconnaissance visuelle par l'utilisateur de la cible qui est actuellement affichée sur le dispositif d'affichage (13), effectue un traitement de rétroaction pendant un temps prescrit pour informer l'utilisateur que la détection s'est produite, ledit temps durant lequel le traitement de rétroaction est effectué étant compris entre 200 ms et 800 ms, ladite unité de rétroaction de détection de reconnaissance visuelle (65) étant de sorte que le traitement de rétroaction vienne à amener la cible, qui est actuellement affichée sur le dispositif d'affichage (13) et qui a été reconnue visuellement, à changer de couleur ou de forme, ladite unité de test de champ visuel (60) étant de sorte que, conjointement avec la conclusion du traitement de rétroaction, la cible qui a été reconnue visuellement soit amenée à disparaître, et la cible suivante soit affichée sur le dispositif d'affichage (13).
